## Europäisches Patentamt

## European Patent Office

## Office européen des brevets

(11) Veröffentlichungsnummer: **0 166 030 B1**

(12) **EUROPÄISCHE PATENTSCHRIFT**

(45) Veröffentlichungstag der Patentschrift:
03.08.88

(21) Anmeldenummer: **84115579.9**

(22) Anmeldetag: **17.12.84**

(51) Int. Cl.⁴: **G 21 K 1/02,** A 61 B 6/02, A 61 B 6/06

(54) Primärstrahlenblende für eine Röntgendiagnostikanlage für Röntgen-Stereobilder.

(30) Priorität: **15.06.84 DE 8422343**

(43) Veröffentlichungstag der Anmeldung:
**02.01.86 Patentblatt 86/1**

(45) Bekanntmachung des Hinweises auf die Patenterteilung:
**03.08.88 Patentblatt 88/31**

(84) Benannte Vertragsstaaten:
**DE FR**

(56) Entgegenhaltungen:
**EP - A - 0 027 291**
**EP - A - 0 074 596**
**DE - A - 2 728 999**
**DE - A - 2 744 808**

(73) Patentinhaber: **Siemens Aktiengesellschaft Berlin und München, Wittelsbacherplatz 2, D-8000 München 2 (DE)**

(72) Erfinder: **Hahn, Alfred, Bunsenstrasse 64, D-8520 Erlangen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft eine Primärstrahlenblende für eine Röntgendiagnostikanlage für Röntgen-Stereobilder mit einem Röntgenstrahler mit zwei im Abstand voneinanderliegenden Fokussen.

Bei einer Röntgendiagnostikanlage dieser Art erfolgt die wechselweise Durchstrahlung des untersuchungsobjektes von jeweils einem Fokus aus. Durch gleichzeitige Betrachtung zweier Bilder, die je einem der Fokusse zugeordnet sind, entsteht für den Betrachter ein räumlicher Bildeindruck. Hinsichtlich der Einblendung der Röntgenstrahlung besteht bei einer Mehrfokus-Röntgenröhre das Problem, für jedes der beiden von den beiden Fokussen ausgesandten Röntgenstrahlenbündel die Durchstrahlung des für die Bildgebung wichtigen Bereiches des Untersuchungsobjektes sicherzustellen, ohne dass mehr Gewebe als erforderlich von Röntgenstrahlung durchdrungen wird.

Der Erfindung liegt die Aufgabe zugrunde, eine Primärstrahlenblende der eingangs genannten Art zu schaffen, bei der jede Strahlungspyramide für jeden Fokus, deren Spitze mit dem jeweiligen Fokus zusammenfällt, optimal einstellbar ist.

Diese Aufgabe ist erfindungsgemäss dadurch gelöst, dass verstellbare Blendenteile vorgesehen sind, mittels derer für jeden Fokus je eine Strahlungspyramide individuell einstellbar ist, und dass die Blendenteile zwei separate verstellbare äussere Blendenöffnungen begrenzen, die zur Einstellung jeweils einer der Strahlenpyramiden dienen.

Aus der EP-A-0 074 596 ist zwar eine Primärstrahlenblende für eine Doppelfokus-Röntgenröhre bekannt, mittels derer für jeden Fokus der Röntgenröhre je eine Strahlungspyramide individuell einstellbar ist, indem die Blendenplatten der Primärstrahlenblende beim Umschalten von dem einen zu dem anderen Fokus synchron in der gleichen Richtung um etwa den gleichen Betrag verschoben werden, um den sich der Fokus verschiebt. Eine solche Lösung kommt für eine Primärstrahlenblende für eine Röntgendiagnostikanlage für Röntgen-Stereobilder jedoch nicht in Frage. Während im Falle einer Doppelfokus-Röntgenröhre nämlich die Fokusse nur in einem sehr geringen Abstand voneinander entfernt angeordnet sind, so dass die erwähnte Verschiebung der Blendenplatten ohne weiteres realisierbar ist, müssen die beiden Fokusse zur Anfertigung von Röntgen-Stereobildern einen Abstand in der Grössenordnung des menschlichen Augenabstandes aufweisen. Eine Verschiebung der Blendenplatten über eine solche Strecke kann aber nicht so rasch erfolgen, wie dies erforderlich ist, um nachteilige Auswirkungen von Bewegungen des Aufnahmeobjektes, z.B. der Organe eines menschlichen Patienten, auf die Abbildungsqualität von Röntgen-Stereobildern zu vermeiden.

Eine zweckmässige Weiterbildung der Erfindung besteht darin, dass zur Einblendung senkrecht zur Stereobasis, also zur Verbindungslinie zwischen den beiden Fokussen, zwischen zwei äusseren Blendenteilen zwei innere, jeweils in Richtung auf das jeweils benachbarte äussere Blendenteil verstellbare Blendenteile vorgesehen sind. Die Begrenzung der beiden Strahlungspyramiden parallel zur Stereobasis kann dabei durch ein für beide Strahlungspyramiden gemeinsames Blendenplattenpaar erfolgen, während für die Begrenzung der beiden Strahlungspyramiden senkrecht zur Stereobasis sowohl die äusseren als auch die inneren Blendenteile verstellt werden können. Die inneren Blendenteile können dabei so ausgebildet sein, dass sie bei ihrer Verstellung nach aussen eine mittlere Strahlungspyramide eines mittleren Fokus freigeben und zusammen mit den äusseren Blendenteilen die äusseren Blendenöffnungen schliessen. Bei dieser Ausbildung ist es möglich, ausser Stereobildern auch Normalbilder mit einem mittleren Fokus anzufertigen und dabei ebenfalls eine optimale Einblendung vorzunehmen.

Die Erfindung ist nachfolgend anhand eines in der Zeichnung dargestellten Ausführungsbeispieles näher erläutert. Es zeigen:

Fig. 1 eine Darstellung einer Primärstrahlenblende nach der Erfindung,

Fig. 2 eine Einzelheit der Primärstrahlenblende gemäss Figur 1, und

Fig. 3 eine Darstellung der Primärstrahlenblende gemäss Figur 1 in einer um 90° verdrehten Ansicht.

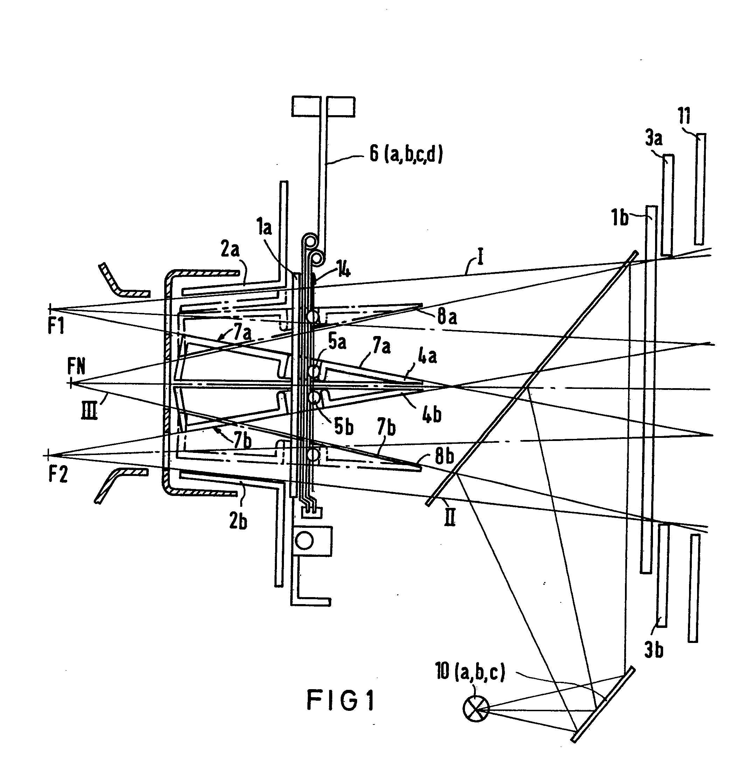

In der Figur 1 ist eine Primärstrahlenblende dargestellt, die die Einblendung von drei von drei Fokussen F1, F2 und FN ausgehenden Strahlungspyramiden erlaubt. Die Fokusse F1 und F2 dienen dabei zur Anfertigung von Stereobildpaaren, während der Fokus FN zur Erzeugung eines normalen Röntgenbildes dient. Die Ausblendung parallel zur Stereobasis, d.h. der Strecke F1–F2, erfolgt wie bei normalen Primärstrahlenblende durch fokusnahe und fokusferne Blendenplattenpaare 1a, 1b. Die Ausblendung quer zur Stereobasis erfolgt bei Stereobetrieb (wechselweise Einschaltung der Fokusse F1, F2) aussen durch die fokusnahen Lamellen 2a für den Fokus F1, 2b für den Fokus F2 und durch die fokusfernen Lamellen 3a und 3b. Die Lamellen 2a, 2b und 3a, 3b sind senkrecht zum jeweiligen Zentralstrahl der eingeblendeten Strahlungspyramide in einer Ebene verstellbar, in der die Stereobasis F1–F2 liegt. Für die Lamellen 2a, 2b ist eine innere Stellung strichpunktiert gezeichnet.

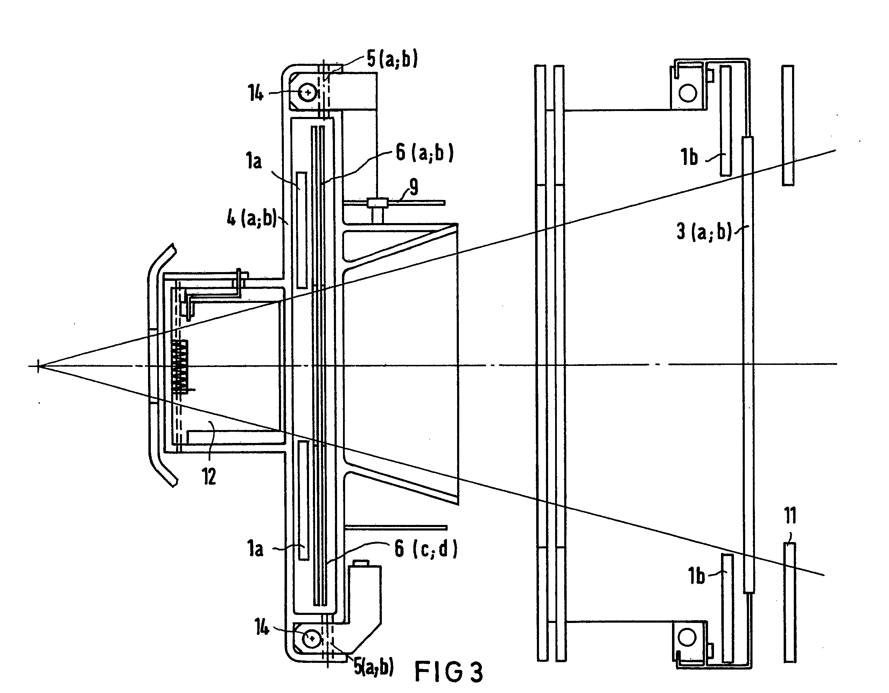

Innen erfolgt die Begrenzung der Strahlungspyramiden durch Schwenkblenden 4a für den Fokus F1 und 4b für den Fokus F2. Die Schwenkblenden 4a, 4b sind um Achsen 5a, 5b schwenkbar und ausserdem parallel zur Stereobasis auf Führungen 14 (Fig. 3) verstellbar. Die Lamellen 2a, 2b, 3a, 3b und die Schwenkblenden 4a, 4b werden durch einen nicht dargestellten Schrittmotor verstellt. Der Bewegungsablauf wird durch einen Mikrocomputer koordiniert.

Für die Anfertigung von Stereobildpaaren nehmen die Teile 1a, 1b, 2a, 2b, 3a, 3b und 4a, 4b ihre voll ausgezogen gezeichneten Stellungen ein, so

dass zwei Strahlungspyramiden I und II einge-blendet werden.

Beim Übergang von Stereo- auf Normalbetrieb koordiniert der Mikrocomputer die Blendenbewegungen zu einer neuen Blendeneinstellung. Die Lamellen 2a, 3a und die Schwenkblende 4a, die bei Stereobetrieb gemäss Figur 1 die Strahlungspyramide I oben und unten begrenzen, bilden bei Normalbetrieb gemeinsam die obere Begrenzung der Strahlungspyramide III des Fokus FN. Entsprechendes gilt für die untere Begrenzung durch die Teile 2b, 3b und 4b.

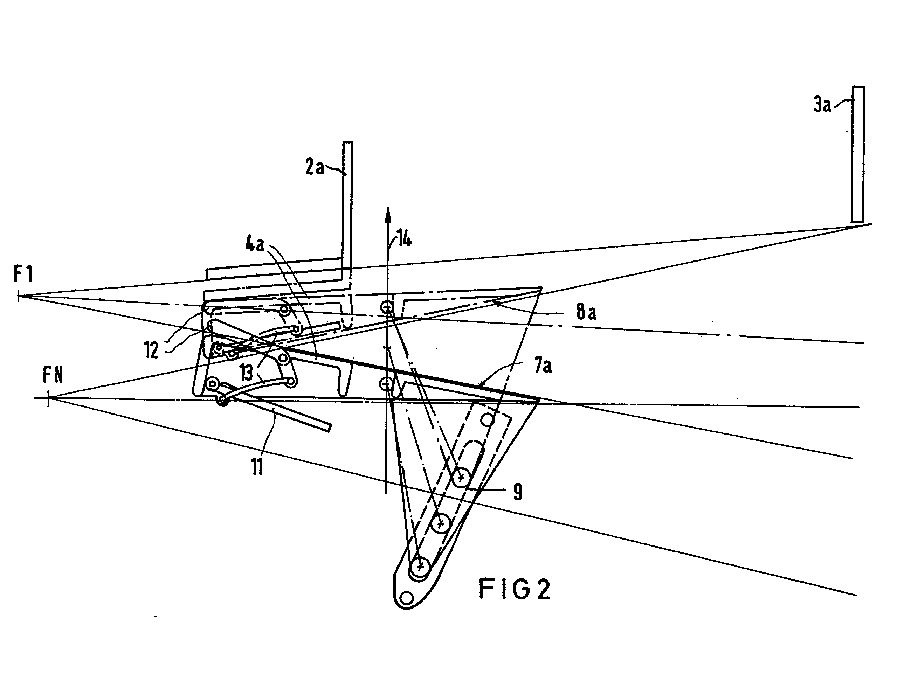

Die Schwenkblenden 4a, 4b sind gemäss Figur 3 so gestaltet, dass in einem Durchbruch im Bereich der Achsen 5a, 5b der Bewegungsraum für die Blendenplattenpaare 1a, 1b und für Filter-schieber 6a, 6b, 6c, 6d frei bleibt. Bei Stereobetrieb wirken die äusseren Begrenzungsflächen 7a, 7b (Figur 2) der Schwenkblenden 4a, 4b strahlungsbegrenzend, bei Normalbetrieb die inneren Begrenzungsflächen 8a, 8b.

Die Schwenkung der Schwenkblenden 4a, 4b erfolgt über eine Kulissenführung 9 gemäss Figur 2.

Im Raum zwischen den Schwenkblenden 4a, 4b und dem Blendenplattenpaar 1b kann gemäss Figur 1 ein Lichtvisier 10a, 10b, 10c, bestehend aus einer Lampe und zwei Spiegeln, angeordnet sein. Für Bildverstärkerbetrieb mit einem Bildver-stärker mit einem kreisförmigen Eingangsleucht-schirm ist auch der Einbau einer Achteckbegrenzung 11 möglich, die in den Figuren nur schematisch dargestellt ist.

Im Raum zwischen den Schwenkblenden 4a, 4b kann eine Schwenkblende 12 gemäss den Figuren 2 und 3 angeordnet sein, die bei Stereobetrieb und kleiner Feldeinblendung, durch Federkraft betätigt, den sich dann öffnenden Spalt zwischen den Schwenkblenden 4a und 4b abdeckt und somit das Austreten von extra-fokaler Strahlung verhindert.

Bei Normalbetrieb wird die Schwenkblende 12 über ein Gestänge 13 von der dann an der Schwenkblende 4a anliegenden Lamelle 2a offen gehalten.

Wesentlich für die Primärstrahlenblende gemäss den Figuren 1 bis 3 ist, dass die Blendenteile 1a, 1b, 2a, 2b, 3a, 3b und 4a, 4b derart ausgebildet und verstellbar sind, dass für jeden Fokus F1, F2 je eine Strahlungspyramide I, II individuell einstellbar ist und dass zur Einblendung senkrecht zur Stereobasis F1, F2 zwischen zwei äusseren Blendenteilen 2a, 2b zwei innere, jeweils in Richtung auf das jeweils benachbarte äussere Blendenteil 2a, 2b verstellbare Blendenteile 4a, 4b vorgesehen sind. Die inneren Blendenteile 4a, 4b sind dabei so ausgebildet, dass sie bei ihrer Verstellung nach aussen eine mittlere Strahlungspyramide III eines mittleren Fokus FN freigeben und zusammen mit den äusseren Blendenteilen 2a, 2b die äusseren Blendenöffnungen für die Strahlungspyramiden I, II schliessen.

Die beschriebene Primärstrahlenblende kann wahlweise als Stereo- oder Normalblende benutzt werden.

**Patentansprüche**

1. Primärstrahlenblende für eine Röntgendiagnostikanlage für Röntgen–Stereobilder mit einem Röntgenstrahler mit zwei Fokussen (F1, F2), dadurch gekennzeichnet, dass verstellbare Blen-denteile (1a, 1b, 2a, 2b, 3a, 3b, 4a, 4b), vorgesehen sind, mittels derer für jeden Fokus (F1, F2) je eine Strahlungspyramide (I, II) individuell einstellbar ist, und dass die Blendenteile (1a, 1b, 2a, 2b, 3a, 3b, 4a, 4b) zwei separate verstellbare äussere Blendenöffnungen begrenzen, die zur Einstellung jeweils einer der Strahlungspyramiden (I, II) dienen.

2. Primärstrahlenblende nach Anspruch 1, dadurch gekennzeichnet, dass zur Einblendung parallel zur Stereobasis (F1, F2) mindestens ein für beide Strahlungspyramiden (I, II) gemeinsames Blendenplattenpaar (1a, 1b) vorgesehen ist.

3. Primärstrahlenblende nach Anspruch 1 oder 2, dadurch gekennzeichnet, dass zur Einblendung senkrecht zur Stereobasis (F1, F2) zwischen zwei äusseren Blendenteilen (2a, 2b) zwei innere, jeweils in Richtung auf das jeweils benachbarte äussere Blendenteil (2a, 2b), verstellbare Blendenteile (4a, 4b) vorgesehen sind.

4. Primärstrahlenblende nach Anspruch 3, dadurch gekennzeichnet, dass die inneren Blendenteile (4a, 4b) so ausgebildet sind, dass sie bei ihrer Verstellung nach aussen eine mittlere Strahlungspyramide (III) eines mittleren Fokus (FN) freigeben und zusammen mit den äusseren Blendenteilen (2a, 2b) die äusseren Blendenöffnungen schliessen.

**Revendications**

1. Diaphragme pour le rayonnement primaire pour une installation de radiodiagnostic servant à réaliser des stéréo-radiographies, comportant un émetteur radiologique possédant deux foyers (F1, F2), caractérisé par le fait qu'il est prévu des élé-ments de diaphragme (1a, 1b, 2a, 2b, 3a, 3b, 4a, 4b) réglables, à l'aide desquels on peut régler individuellement une pyramide de rayonnement (I, II) pour chaque foyer (F1, F2), et que les élé-ments de diaphragme (1a, 1b, 2a, 2b, 3a, 3b, 4a, 4b) délimitent deux ouvertures extérieures du diaphragme, réglables séparément, qui servent à régler respectivement l'une des pyramides de rayonnement (I, II).

2. Diaphragme pour les rayonnements primaires suivant la revendication 1, caractérisé par le fait que pour réaliser une collimation parallèlement à la base stéréo (F1, F2), il est prévu au moins un couple (1a, 1b) de plaques collimatri-ces, commun pour les deux pyramides de rayon-nement (I, II).

3. Diaphragme pour le rayonnement primaire suivant la revendication 1 ou 2, caractérisé par le fait que pour réaliser la collimation perpendiculairement à la base stéréo (F1, F2) il est prévu, entre deux éléments extérieurs de diaphragme (2a, 2b), deux éléments intérieurs de diaphragme (4a, 4b), réglables respectivement en direction des éléments extérieurs de diaphragme (2a, 2b) respectivement voisins.

4. Diaphragme pour le rayonnement primaire suivant la revendication 3, caractérisé par le fait que les éléments intérieurs de diaphragme (4a, 4b) sont agencés de telle sorte que, lors de leur déplacement vers l'extérieur, ils libèrent une pyramide centrale de rayonnement (III) issue d'un foyer central (FN) et délimitent, conjointement avec les éléments extérieurs de diaphragme (2a, 2b), les ouvertures extérieures du diaphragme.

## Claims

1. Primary radiation screen for an X-ray diagnostics installation for producing X-ray stereo images with an X-ray tube having two focal points (F1, F2), characterised in that adjustable screen sections (1a, 1b, 2a, 2b, 3a, 3b, 4a, 4b) are provided, by means of which radiation cones (I, II) can be individually controlled respectively for each focal point (F1, F2), and in that the screen sections (1a, 1b, 2a, 2b, 3a, 3b, 4a, 4b) delimit two separate adjustable outer screen apertures, which serve to control respective ones of the radiation cones (I, II).

2. Primary radiation screen according to claim 1, characterised in that at least one pair of screen plates (1a, 1b) is provided in common for both radiation cones (I, II) for focusing parallel to the stereo base (F1, F2).

3. Primary radiation screen according to claim 1 or 2, characterised in that there are provided between two outer screen sections (2a, 2b) for focusing perpendicular to the stereo base (F1, F2) two inner adjustable screen sections (4a, 4b), which are directed towards the respectively adjacent outer screen sections (2a, 2b).

4. Primary radiation screen according to claim 3, characterised in that the inner screen sections (4a, 4b) are designed in such a way that when they are displaced to the outside, they allow through a central radiation cone (III) of a central focal point (FN) and, together with the outer screen sections (2a, 2b), close the outer screen apertures.

FIG 1

FIG 2

FIG 3